# FASCICULE DE BREVET EUROPEEN

(11) **EP 1 236 433 B1**
(45) Date de publication et mention de la délivrance du brevet: **29.11.2006**
(21) Numéro de dépôt: 01105076.2
(22) Date de dépôt: 02.03.2001
(51) Int. Cl.: A61B 3/032, A61B 5/00, A61B 5/12

(54) **Procédé de production de tests ou d'exercices visuels ou auditifs d'enregistrement des réactions du sujet et d'analyse des résultats**
Verfahren zur Erzeugung von visuellen oder auditiven Testen oder Übungen, Registrierung und Analyse der Ergebnisse
Process of production of visual or auditive tests or exercices, recording the subject's reactions and analysis of the results

(43) Date de publication de la demande: 04.09.2002
(73) Titulaire: Ledent, Luc, 4317 Les Waleffes (BE)
(72) Inventeur: Ledent, Luc, 4317 Les Waleffes (BE)

(56) Documents cités:
- WO-A-98/49930
- US-A- 4 847 763
- US-A- 5 550 602
- US-A- 5 920 375
- US-A- 5 953 102
- US-A- 6 045 227

## Description

### Domaine technique :

Le domaine de l'invention est celui de l'évaluation de la vision ou de l'audition d'un individu humain ou animal. Plus précisément, l'invention concerne un procédé d'évaluation utilisant un matériel de commande et de gestion informatique qui pilote un ou plusieurs écrans de visualisation, une ou plusieurs sources sonores, une caméra d'enregistrement vidéo et, éventuellement en plus, un micro pour l'enregistrement sonore et un périphérique de pointage de type souris ou joystick manipulé par le sujet. De plus, les moyens classiques de support de verres correcteurs, caches, filtres, verres colorés ou polarisés ou autre système correctif ou discriminatif de la vue et les moyens correctifs ou discriminatifs de l'audition peuvent également être utilisés.

### Etat de la technique antérieure :

Il existe actuellement une panoplie de méthodes d'exploration de la fonction visuelle ou auditive.

Voici quelques exemples de méthodes existantes d'exploration de la fonction visuelle :
- Etude de l'acuité visuelle de loin :
   Projection lumineuse de symboles sur un support placé à une distance prédéterminée. On citera par exemple les anneaux brisés de Landolt ou l'échelle de Monoyer : présentation de groupes de lettres ou de chiffres d'une dimension particulière correspondant à un degré de l'échelle de vision (calculée pour un distance de 5 mètres).
- Etude de l'acuité visuelle de près :
   On utilise notamment l'échelle de Parinaud qui propose la lecture d'un texte dont la taille des caractères varie. Ce test évalue plutôt les capacités lexicales du sujet que son acuité.
- Etude de l'acuité visuelle de loin et de près chez l'enfant :
   On emploie la reproduction de figurines familières dont le tracé est choisi empiriquement pour s'approcher du résultat donné par l'anneau brisé de Landolt (échelle de Rossano, de Pigassou, de Sander-Zanlonghi).
- Etude de la vision des couleurs :
   Les équations colorées font comparer un mélange coloré variable à une couleur de référence invariable (colorimètre à canaux optiques). Une autre méthode fait déchiffrer un signe d'une couleur donnée sur un fond d'une autre couleur (tableau d'Ishihara), ou classer des pions colorés par ordre de similitude (FM 100-hue, Panel D-15).
- Etude de la vision aux basses et hautes luminances : éblouissement et adaptométrie.
- Etude de la vision binoculaire : tests de Worth, de Bagolini.
- Etude de la vision stéréoscopique : tests Titmus, TNO, Lang.
- Etude des mouvements d'attraction visuelle et de poursuite chez le jeune enfant :
   Méthode du regard préférentiel (cartons de Teller, Bébé Vision Tropique), méthode du nystagmus optocinétique (tambour tournant sur lequel figurent des mires : bandes noires et blanches verticales ou des dessins), méthode de la poursuite visuelle d'une mire structurée en mouvement.
- Méthodes électro-physiologiques.
- Méthode utilisant des moyens informatiques avec visualisation de tests calibrés sur un écran (Brevet EP0925012 - WO9849930).

Parmi toutes les méthodes existantes, aucune ne propose à la fois toutes les caractéristiques définies dans la présentation de l'invention.

### Présentation de l'invention :

Dans l'évaluation de la vision, le ou les écrans de visualisation présentent un test ou un exercice visuel et dans le même temps, la caméra et éventuellement le micro et le périphérique de pointage enregistrent les réactions du patient. La ou les sources sonores peuvent servir à donner des instructions, poser des questions ou produire un bruitage destiné à désigner des objets ou à attirer l'attention du sujet vers le test visuel.

Dans l'évaluation de l'audition, la ou les sources sonores produisent un test ou un exercice auditif et dans le même temps, la caméra et éventuellement le micro et le périphérique de pointage enregistrent les réactions du patient. Le ou les écrans de visualisation peuvent servir à afficher des instructions, à poser des questions, à désigner des objets correspondant aux sons entendus ou à attirer l'attention du sujet (vers la caméra par exemple).

L'ensemble de tous les éléments n'est pas nécessairement requis pour caractériser l'invention. Par exemple, les enregistrements via le micro et le périphérique de pointage peuvent ne pas être utilisés.

La procédure d'évaluation ou de stimulation de l'audition ou de la vision se déroule en plusieurs phases :
- Phase de paramétrage : ajustement des paramètres variables et définition des scénarios de tests ou d'exercices pour les différents publics cibles.
- Phase de test ou d'exercice : réalisation des tests ou des exercices avec le sujet comprenant l'enregistrement synchronisé des réactions du sujet, la diffusion éventuelle d'un commentaire sonore ou visuel synchronisé et l'enregistrement éventuel des résultats observés en cours de réalisation.
- Phase d'analyse : analyse à postériori par l'observateur ou par un autre clinicien grâce aux moyens de revisualisation synchronisée des tests ou des exercices et des réactions (vidéo et/ou sonore et/ou de pointage) du sujet en vitesse réelle, accélérée, ralentie, pas à pas ou en mode pause.

Cette analyse permet de détecter très précisément à quel stade du test le sujet réagit et de quelle façon, de chronométrer le temps de latence entre la présentation d'un objet ou d'un son et sa reconnaissance.

Les réactions observées peuvent être par exemple et de manière non exhaustive :
- Les mouvements de yeux.
- Les mouvements de la tête.
- Les mouvements du cou et du tronc (par exemple : rapprochement ou éloignement de l'écran ou de la source sonore).
- Les mouvements des bras et des mains.
- La dilatation ou la contraction pupillaire.
- L'expression du visage.
- Les réponses aux questions.
- Les citations d'objets, de bruits, de lettres ou de chiffres.
- La lecture ou la répétition de mots ou de textes.
- Le pointage d'objets ou d'images.
- L'attitude générale du sujet et notamment ses capacités d'attention.

Les tests et les exercices visuels et auditifs produits peuvent être très variés et très nombreux. Ils peuvent présenter des images ou des sons statiques ou en mouvement.

Voici quelques exemples de paramètres pouvant être modulés avec les tests et les exercices visuels :
- Taille des objets ou des symboles.
- Contraste lumineux.
- Couleurs.
- Durée et fréquence d'apparition des objets ou des symboles.
- Textes présentés à la lecture : dimension et espacement des caractères et des lignes, contenu et langue du texte.
- Vitesse de déplacement et trajectoire des objets ou des symboles.
- Image de fond : inexistante, fixe ou animée.
- OEil testé : le même test pour les deux yeux simultanément, un oeil à la fois (cache ou filtre sur l'autre oeil), un test différent pour chaque oeil (deux images de visualisation et un moyen discriminatif pour que chaque oeil voie une seule image).
- Décalage des images dans les tests de vision stéréoscopique.

Voici quelques exemples de paramètres pouvant être modulés avec les tests et les exercices auditifs :
- Intensité, fréquence et durée des sons (audiométrie tonale).
- Séquençage de sons distincts, uniformes, modulés ou enchaînés.
- Mots prononcés : variation de l'intensité, du vocabulaire et de la langue (audiométrie vocale).
- Déplacement des sons (stéréophonie).
- Bruitage de fond : absent, uniforme ou modulé.
- Test des deux oreilles simultanément, d'une oreille à la fois ou test différent pour chaque oreille (casque stéréophonique).

Toujours à titre d'exemple, voici quelques tests classiques pouvant être produits par le système :
- Etude de l'acuité visuelle de loin.
- Etude de l'acuité visuelle de près.
- Etude de l'acuité visuelle du bébé.
- Etude de la vision des couleurs.
- Etude de la vision stéréoscopique (avec verres colorés ou polarisés).
- Etude de la vision binoculaire.
- Etude de la vision nocturne (vision mésopique et scotopique) avec utilisation possible d'une caméra infra-rouge.
- Etude des réflexes pupillaires.
- Audiométrie tonale et vocale de l'adulte.
- Audiométrie tonale et vocale de l'enfant.
- Etude des réflexes d'orientation (mouvement des globes oculaires et de la tête vers la source sonore) du bébé et de l'enfant.
- Recherche du recrutement (test auditif de Fowler).
- Etude de réflexes moteurs ou de réactions motrices d'origine visuelle ou auditive.

Un premier avantage de l'invention est qu'elle permet de produire une très grande variété de tests et d'exercices et notamment des tests qui correspondent à des situations de perception sensorielle plus réelles que les tests actuellement utilisés. L'observateur a lui-même la possibilité de modifier les tests en faisant varier certains paramètres. Il va de soi qu'une expérimentation clinique et diverses standardisations effectuées par les cliniciens du domaine avec le procédé inventé pourra et devra avoir lieu. Mais même avant cela, le procédé de l'invention a déjà une grande valeur en ce sens qu'il complète et affine les mesures effectuées avec les tests classiques.

Un deuxième avantage de l'invention est qu'elle mémorise non seulement le résultat final du test mais aussi les réactions du patient pendant le test ce qui augmente l'objectivité de l'examen et permet par la suite de comparer plus finement plusieurs examens ou de soumettre l'analyse des examens à plusieurs observateurs différents.

Un troisième avantage de l'invention est que la participation volontaire du patient n'est pas toujours nécessaire. On peut donc utiliser l'invention avec de très jeunes enfants, des personnes à capacités réduites ou avec des animaux en combinant les stimuli visuels et auditifs et en étudiant des paramètres tels que les mouvements des yeux ou de la tête ou la dilatation ou la contraction pupillaire.

Un quatrième avantage de l'invention est que des séquences de tests (scénarios) peuvent être programmées et utilisées en fonction des catégories cliniques et en fonction des réponses du sujet.

Un cinquième avantage de l'invention est que les instructions données au sujet avant et pendant le test peuvent être prédéfinies, enregistrées et diffusées de façon synchrone avec le test. Ceci augmente l'objectivité du test en diminuant le pouvoir suggestif de l'examinateur. Ceci permet également l'utilisation du test dans différentes langues y compris des langues peu ou pas maîtrisées par l'observateur.

Un sixième avantage de l'invention est que la vue aide à tester l'ouïe et vice-versa. Un enfant peut montrer l'image d'un animal dont il entend le cri ou préciser si la trajectoire d'une ambulance qu'il voit à l'écran correspond ou non à la trajectoire stéréophonique de la sirène qu'il entend.

Un septième avantage de l'invention est qu'elle peut servir à produire des exercices ludiques (à la façon des jeux vidéo) de rééducation de la vue et de l'ouïe, d'en contrôler l'exécution et les résultats.

Un huitième avantage de l'invention est qu'elle est évolutive puisque avec le même matériel et en adaptant le logiciel, on peut produire de nouveaux tests ou de nouveaux exercices et répondre ainsi à la demande des utilisateurs.

Un neuvième avantage de l'invention est que plusieurs tests peuvent être utilisés avec le même matériel sans déplacement du patient ni de l'observateur avec en conséquence un gain de temps.

Un dixième avantage de l'invention est qu'elle ne requiert pas un matériel médical spécifique. La plupart des éléments matériels de l'invention sont commercialisés à grande échelle.

Un onzième avantage de l'invention est qu'elle peut imprimer via une imprimante connectée au système informatique tous les résultats des tests sous forme de tableaux et de graphiques.

Un douzième avantage de l'invention est qu'elle peut être facilement connectée au dossier médical informatisé du patient.

### Mode de réalisation :

D'autres caractéristiques et avantages de l'invention apparaîtront plus clairement à la lecture de la description suivante de deux modes de réalisation et d'un exemple d'utilisation, donnés à titre de simples exemples illustratifs et non limitatifs et des dessins annexés, parmi lesquels :
- la figure 1 illustre un mode de réalisation simplifié de l'invention ;
- la figure 2 illustre un mode de réalisation plus complet de l'invention ;
- la figure 3 illustre un exemple très simplifié d'utilisation.

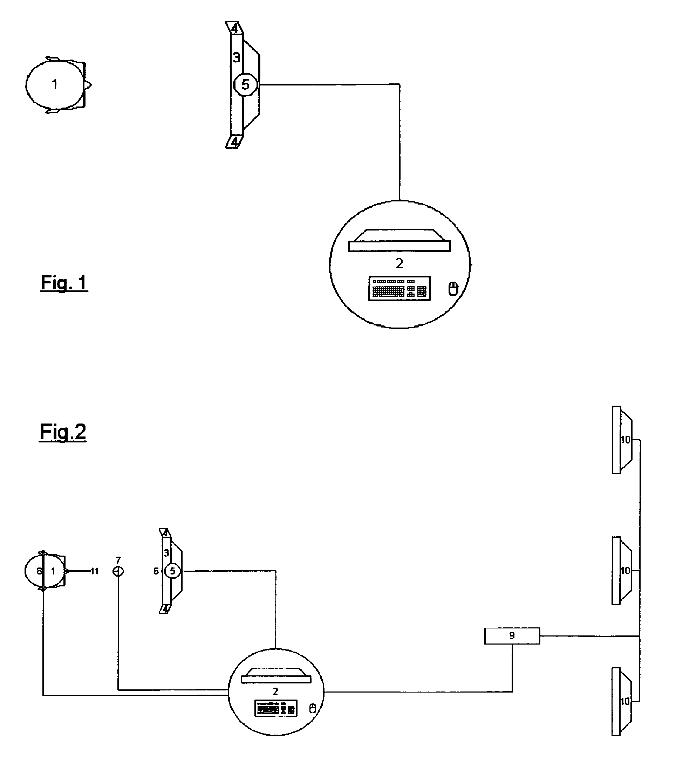

### Mode de réalisation simplifié de l'invention (Figure 1).

- 1: Sujet avec ou sans verres correcteurs ou avec ou sans système discriminatif de la vue devant les yeux.
- 2: Poste de commande informatique.
- 3: Ecran de visualisation de près.
- 4: Haut-parleurs.
- 5: Caméra.

Le poste de commande informatique (2) manipulé par l'observateur démarre un test ou un exercice visuel ou auditif qui est produit par l'écran de visualisation (3) et/ou les sources sonores (4) et, de façon synchrone, il démarre l'enregistrement des réactions du sujet (1) par la caméra (5). Le sujet (1) voit sur l'écran de visualisation (3) uniquement la partie visuelle du test, tandis que l'observateur voit sur l'écran du poste de commande (2) l'interface du logiciel qui pilote le test. L'observateur peut ainsi voir en direct à la fois le test et les réactions enregistrées. Après le test, l'observateur peut revoir le test et en synchronisation les réactions enregistrées soit à la vitesse réelle, soit au ralenti, soit en mode arrêt sur image.

### Mode de réalisation plus complet de l'invention (Figure 2).

- 1 à 5: Identiques à la figure 1.
- 6: Microphone.
- 7: Dispositif de pointage manipulé par le sujet.
- 8: Casque stéréophonique ou vibrateur sur les mastoïdes.
- 9: Matériel informatique de contrôle du ou des écrans de visualisation de loin.
- 10: Ecran(s) de visualisation de loin.
- 11: Ecran discriminatif opaque de séparation des images vues par chaque oeil.

Le mode de réalisation plus complet de l'invention part du même principe que le mode simplifié décrit avec la figure 1, mais il ajoute les possibilités suivantes :
- utilisation d'une source sonore supplémentaire : casque stéréophonique ou vibrateur mastoïdien (8) ;
- utilisation d'un microphone (6) pour enregistrer les réactions sonores du sujet (1). Le microphone (6) peut être indépendant ou couplé à l'écran (3), à la caméra (5) ou au casque stéréophonique (8).
- utilisation soit de l'écran de visualisation de près (3), soit de un, deux ou trois écrans de visualisation de loin (10) pilotés ou non par un matériel de contrôle informatique supplémentaire (9). Les images sur les écrans de loin (10) peuvent être semblables ou différentes.
- utilisation d'un dispositif de pointage (7) de type souris ou joystick manipulé par le sujet pour pointer des éléments visuels du test sur un des écrans de visualisation (3) ou (10).
- utilisation d'un écran opaque discriminatif (11) installé sur la tête du sujet (1) pour séparer les images vues par chaque oeil.

Il va de soi que ces possibilités supplémentaires ne seront pas toutes utilisées simultanément, mais plutôt choisies en fonction de la spécificité du test ou de l'exercice produit.

La disposition pratique du matériel peut se faire, entre autres, de la manière suivante :
- les périphériques de près : écran (3), haut-parleurs (4), caméra (5) et périphérique de pointage (7) peuvent être regroupés sur une table à roulettes réglable en hauteur (à des hauteurs avoisinant les 75 cm) ou fixés avec un système de réglage de la distance par rapport au sujet (1) (entre 30 et 90 cm) sur une tablette coulissant devant le sujet (1), lui-même assis sur un siège réglable en hauteur ;
- les périphériques de loin : écrans (10) peuvent être fixés au moyen de supports muraux ou sur pieds à une hauteur avoisinant 180 cm et à une distance de 3 à 6 mètres du sujet (1).

Selon cette disposition, la caméra (5) est située juste au-dessus du regard vers les périphériques de près (3) et juste au-dessous du regard vers les périphériques de loin (10).

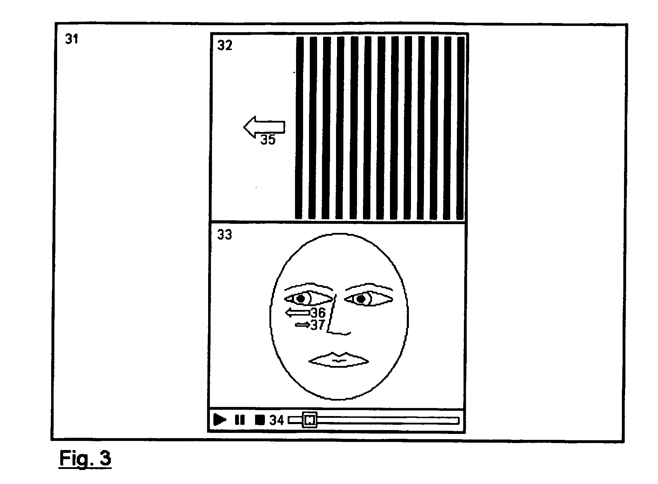

### Exemple simplifié d'utilisation de l'invention (Figure 3).

La figure 3 montre l'écran (31) du poste de commande informatique (2) représenté sur les figures 1 et 2. On y voit deux fenêtres de visualisation (32) et (33) et une barre de contrôle chronologique du défilement (34). Les flèches (35), (36) et (37) seront décrites avec l'explication de l'exemple.

L'exemple simplifié décrit la réalisation d'un test utilisant la méthode du nystagmus optocinétique. Le nystagmus optocinétique se caractérise par une série de mouvements oculaires réflexes se produisant de façon répétée chez un sujet qui regarde une succession d'objets défilant rapidement. Ce test est couramment utilisé pour donner une information globale sur la présence ou l'absence de la fonction visuelle chez un sujet non coopérant ou chez un enfant.

Pour réaliser ce test, on se base sur le mode de réalisation simplifié de l'invention (figure 1). On fait démarrer le test sur l'écran de visualisation (3) par quelques clignotements d'un objet (par exemple une étoile) situé au centre de l'écran pour attirer le regard du sujet vers le centre de l'écran. Dans le même temps, on démarre l'enregistrement des réactions du sujet (1) via la caméra (5). Le test produit ensuite un défilement à une vitesse donnée de bandes verticales noires et blanches par exemple de la gauche vers la droite de l'écran (3). Après quelques secondes, le défilement s'interrompt et le test est terminé pour le sujet.
La figure 3 montre l'analyse du test par l'observateur. Les fenêtres de visualisation (32) et (33) sont synchronisées. L'observateur voit en même temps les évènements du test (défilement des bandes (35)) et les réactions du patient (36 et 37). Dans le cas de l'exemple, on observe la succession de deux types de mouvements oculaires chez le sujet (1) : un premier mouvement de poursuite lent et uniforme (36) dans le sens du déplacement des bandes, un deuxième mouvement sous forme de saccades (37) qui ramène brutalement les yeux à leur position de départ. La barre de contrôle chronologique du défilement (34) permet l'analyse du test en vitesse réelle, accélérée, ralentie, en mode pause ou pas à pas. Cette barre de contrôle peut être plus élaborée que la représentation sur la figure.

Malgré l'extrême simplicité de l'image construite pour ce test, on peut faire varier plusieurs facteurs :
- sens et vitesse du défilement ;
- largeur des bandes (le pouvoir séparateur de l'oeil du sujet équivaut à la valeur angulaire du plus petit test qui va produire un nystagmus optocinétique) ;
- couleur des bandes (test de la vision des couleurs).

## Revendications

1. Procédé d'évaluation de la vision ou de l'audition d'un individu humain ou animal ; ce procédé affiche pour tester la vision des objets, des symboles, des séquences d'images sur un ou plusieurs écrans de visualisation (3,10) et sur un écran de contrôle (2) ; ce procédé diffuse pour tester l'audition des sons, des séquences sonores, des commentaires par une ou plusieurs sources sonores (4,8) ; ce procédé enregistre les réactions du sujet (1) par une caméra (5) et éventuellement par un microphone (6) et un périphérique de pointage (7); le tout est piloté par un système informatique (2,9).

2. Procédé selon la revendication 1, **caractérisé en ce qu'**il comprend les phases suivantes :
• phase de paramétrage : ajustement au moyen du logiciel et du poste de contrôle informatique (2) des paramètres variables des tests et des exercices et définition des scénarios de tests et des scénarios d'exercices selon les publics cibles et selon les réponses du sujet (1) ;
• phase de test ou d'exercice : réalisation des tests ou des exercices avec le sujet (1) comprenant l'enregistrement synchronisé des réactions du sujet (5,6,7), la diffusion d'un commentaire sonore (4,8) ou visuel (3,10) synchronisé et l'enregistrement avec le poste de contrôle informatique (2) des résultats observés en cours de réalisation ;
• phase d'analyse : analyse à postériori des résultats par l'observateur ou par un autre clinicien avec les moyens de revisualisation synchronisée des tests et des exercices et des réactions du sujet (1) en vitesse réelle, accélérée, ralentie, pas à pas ou en mode pause sur le poste de contrôle informatique(2,31,32,33,34).

3. Procédé selon la revendication 2, **caractérisé en ce que** ladite phase de paramétrage agit sur les paramètres suivants :
• la taille des objets et des symboles visuels ;
• le contraste lumineux ;
• les couleurs ;
• la durée et la fréquence d'apparition des objets et des symboles ;
• les caractères ou les textes présentés à la lecture : dimension et espacement des caractères et des lignes, contenu ou langue du texte ;
• la vitesse de déplacement et la trajectoire des objets et des symboles ;
• l'image de fond : inexistante, fixe ou animée ;
• le décalage des images dans les tests de vision stéréoscopique ;
• l'intensité, la fréquence et la durée des sons ;
• le séquençage de sons distincts, uniformes, modulés ou enchaînés ;
• les mots prononcés : variation de l'intensité, du vocabulaire et de la langue ;
• le déplacement des sons : stéréophonie ;
• le bruitage de fond : absent, uniforme ou modulé ;
• les commentaires sonores et visuels préenregistrés : instructions, questions et bruitages ainsi que la langue de ces commentaires.

4. Procédé selon la revendication 2, **caractérisé en ce que** ladite phase de test ou d'exercice concerne :
• les deux yeux simultanément avec la même séquence d'images ;
• les deux yeux simultanément avec une séquence d'images différente pour chaque oeil en utilisant par exemple un moyen discriminatif (11) pour séparer les images vues par chaque oeil;
• un oeil à la fois avec cache devant l'autre oeil ;
• les deux oreilles simultanément avec la même séquence sonore ;
• les deux oreilles simultanément avec une séquence sonore différente pour chaque oreille par utilisation du casque stéréophonique ou du vibreur mastoïdien (8) ;
• une oreille à la fois par utilisation du casque stéréophonique ou du vibreur mastoïdien (8) ;
• la vue et l'ouïe indépendamment ou simultanément : l'étude ou la stimulation de l'ouïe s'appuyant sur les perceptions ou les réflexes visuels et vice-versa ; l'ouïe pouvant attirer l'attention vers un test visuel ou vice-versa.

5. Procédé selon les revendications 2 et 4, **caractérisé en ce que** ladite phase de test ou d'exercice s'applique aux domaines suivants :
• la fonction visuelle ;
• la fonction auditive ;
• l'acuité visuelle de loin ;
• l'acuité visuelle de près ;
• l'acuité visuelle du bébé ;
• la vision des couleurs ;
• la vision stéréoscopique avec verres colorés ou polarisés ;
• la vision binoculaire ;
• la vision nocturne avec utilisation possible d'une caméra infra-rouge ;
• l'étude des réflexes pupillaires ;
• l'audiométrie tonale et vocale de l'adulte ;
• l'audiométrie tonale et vocale de l'enfant ;
• l'étude des réflexes d'orientation du bébé et de l'enfant par observation des mouvements des globes oculaires et de la tête vers la source sonore ;
• la recherche du recrutement par exemple par le test auditif de Fowler ;
• l'étude de réflexes moteurs et de réactions motrices d'origine visuelle ou auditive.

6. Procédé selon la revendication 2, **caractérisé en ce que** ladite phase d'analyse permet l'observation et l'analyse chronologique en référence synchronisée aux tests ou aux exercices des réactions suivantes du sujet (1) :
• les mouvements des yeux ;
• les mouvements de la tête ;
• les mouvements du cou et du tronc ;
• les mouvements des bras et des mains ;
• la dilatation ou la contraction pupillaire ;
• l'expression du visage ;
• les réponses aux questions ;
• les citations d'objets, de bruits, de lettres ou de chiffres ;
• la lecture ou la répétition de mots ou de textes ;
• le pointage d'objets ou d'images ;
• l'attitude générale du sujet (1) et notamment ses capacités d'attention.

7. Dispositif de mise en oeuvre du procédé selon l'une quelconque des revendications 1 à 6, **caractérisé par** la disposition suivante du matériel :
• les périphériques de près : écran (3), haut-parleurs (4), caméra (5), microphone facultatif (6) et périphérique de pointage facultatif (7) sont regroupés sur une table à roulettes réglable en hauteur à des hauteurs avoisinant les 75 cm ou fixés avec un système de réglage de la distance par rapport au sujet (1) entre 30 et 90 cm sur une tablette coulissant devant le sujet (1), lui-même assis sur un siège réglable en hauteur avec en variante les haut-parleurs (4) et le microphone (6) regroupés sur un casque (8) porté par le sujet (1) ;
• les périphériques de loin sont facultatifs : les écrans (10) sont fixés au moyen de supports muraux ou sur pieds à une hauteur avoisinant 180 cm et à une distance de 3 à 6 mètres du sujet (1) ;
disposition selon laquelle, la caméra (5) est située juste au-dessus du regard vers les périphériques de près (3) et juste au-dessous du regard vers les périphériques de loin facultatifs (10).

## Claims

1. Process of visual or hearing assessment of an individual or an animal; in order to test visual perception this process displays objects, symbols, image sequences on one or several display screens (3,10) and on one picture monitor (2) ; in order to test hearing ability this process displays sounds, sound sequences, comments through one or several sound sources (4,8) ; this process records the subject's reactions (1) with a camera (5) and if need be with a microphone (6) and a pointing device (7) ; this all is run by an information processing system (2,9).

2. Process according to claim 1, **characterised by** the following phases :
• parameterization phase : adjustment with the software and the computer-based system (2) of the variable parameters of the tests and exercises and definition of the test and exercise scenarios according to the target publics and the subject's answers (1) ;
• test or exercise phase: realisation of tests and exercises with the subject (1) including the synchronised recording of the subject's reactions (5,6,7), the broadcasting of a synchronised (4,8) or visual comment (3, 10) and the recording with the computer-based system (2) of the results observed during realisation ;
• analysis phase : a posteriori analysis of the results by the observer or another clinician with the means of synchronised revisualization means of the tests, exercises and subject's reactions (1) at actual, accelerated, idling speed, step by step or in pause mode on the computer-based system (2,31,32,33,34).

3. Process according to claim 2, **characterised by** the fact that the parameterization phase has an effect on the following parameters:
• the size of objects and visual symbols;
• light contrast;
• colors;
• display duration and frequency of objects and symbols;
• characters or texts submitted for reading : character size and spacing, text's content and language ;
• displacement velocity and trajectory of objects and symbols;
• background picture: no picture, still or motion picture;
• image shifting in the stereoscopic vision tests ;
• intensity, frequency and duration of sounds ;
• sequencing of distinct, uniform, modulated sounds or sounds following one another ;
• the words pronounced : variation in intensity, vocabulary and language;
• sound shifting : stereophony ;
• background sounds: no sounds, uniform or modulated sounds;
• prerecorded sound and visual comments: instructions, questions, sound effects and the language of these comments.

4. Process according to claim 2, **characterised by** the fact that the test or exercise phase concerns:
• both eyes simultaneously with the same image sequence;
• both eyes simultaneously with a different image sequence for each eye for example by using a discriminative mean (11) to separate the pictures seen by each eye;
• one eye at a time while the other eye is covered ;
• both ears simultaneously with the same sound sequence ;
• both ears simultaneously with a different sound sequence for each ear by using stereophonic earphones or a mastoid vibrator (8) ;
• one ear at a time by using stereophonic earphones or a mastoid vibrator (8) ;
• visual perception and hearing ability separately or simultaneously : testing or stimulating hearing ability on the basis of perceptions or visual reflexes and vice versa ; considering that hearing ability can draw the attention on a visual test or vice versa.

5. Process according to claims 2 and 4, **characterised by** the fact that the test or exercise phase applies to the following fields:
• visual function ;
• hearing function ;
• far visual acuity;
• near visual acuity;
• visual acuity of the baby;
• color vision;
• stereoscopic vision with colored or polarized glasses ;
• binocular vision;
• night vision with possible use of an infrared camera ;
• testing of pupillary reflexes ;
• tonal and vocal audiometry of adult patients;
• tonal and vocal audiometry of infant patients;
• testing of the baby and infant's orientation reflex by observing the movements of the eyeballs and the head towards the sound source ;
• identification of loudness recruitment for example with the hearing test of Fowler ;
• testing of motor reflexes and visual or auditory motor reactions.

6. Process according to claim 2, **characterised by** the fact that the analysis phase enables chronological observation and analysis with synchronised reference to the tests and exercises of the subject's (1) following reactions:
• eye movements ;
• head movements ;
• neck and trunk movements;
• arm and hand movements ;
• pupillary dilatation or contraction;
• face expression;
• answers to questions;
• mention of objects, noises, letters or numbers;
• reading or repetition of words or texts;
• pointing to objects or pictures ;
• the subject's general attitude (1) and particularly its attention abilities.

7. Implementation system of the process according to any claim from claim 1 to claim 6, **characterised by** the following equipment arrangement:
• peripheral devices in close range : the screen (3), the loudspeakers (4), the camera (5), the optional microphone (6) and the optional pointing device (7) are put together on a height-adjustable rolling table of about 75 cm high or are fixed with a remote control system at a distance between 30 and 90 cm from the subject (1) on a sliding board in front of the subject (1), who is himself seated on a height-adjustable seat with alternatively the loudspeakers (4) and the microphone (6) grouped together on earphones (8) worn by the subject (1) ;
• the remote peripheral devices are optional : screens (10) are fixed with wall or stand holders at a height of about 180 cm and a distance of 3 to 6 meters from the subject (1) ;
according to this arrangement the camera (5) is situated just above the eyes towards the peripheral devices in close range (3) and just below the optional remote peripheral devices (10).

## Patentansprüche

1. Verfahren zur Einschätzung der Sehfähigkeit oder des Gehörs eines Menschen oder eines Tieres; zur Überprüfung der Sehfähigkeit gibt dieses Verfahren Gegenstände, Symbole, Bildsequenzen auf ein oder mehrere Bildschirme (3,10) und auf einen Kontrollschirm (2) aus; zur Überprüfung des Gehörs gibt dieses Verfahren Töne, Schallsequenzen, Kommentare auf eine oder mehrere Schallquellen (4,8) aus; die Reaktionen des Patienten (1) werden durch dieses Verfahren mit einer Kamera (5) und gegebenenfalls mit einem Mikrophon (6) und einem Zeigegerät (7) registriert; das alles wird durch ein Datenverarbeitungssystem (2,9) gesteuert.

2. Verfahren nach dem Patentanspruch unter 1., das durch folgende Phasen **gekennzeichnet** ist :
• Parametrisierungsphase: Anpassung, mit der Software und dem Computersystem (2) den veränderlichen Parameter der Tests und der Übungen und Festlegung der Test- und Übungsszenarien gemäß den Zielpubliken und den Antworten des Patienten (1) ;
• Test- oder Übungsphase: Ausführung von Tests und Übungen an dem Patienten (1) einschließlich der synchronisierten Registrierung der Reaktionen des Patienten (5,6,7), die Verbreitung eines synchronisierten akustischen (4,8) oder visuellen Kommentares (3,10) und Registrierung mit dem Computersystems (2) der zur Zeit laufenden beobachteten Ergebnisse;
• Analysephase: nachträgliche Analyse der Ergebnisse durch den Beobachter oder einen anderen Kliniker mittels einer synchronisierten Wiederausgabe der Tests, der Übungen und der Reaktionen des Patienten (1) auf eine effektive, beschleunigte, langsamere und allmähliche Geschwindigkeit oder im Pausemode auf das Computersystem (2,31,32,33,34).

3. Verfahren nach dem Patentanspruch 2, das **dadurch gekennzeichnet ist, daß** diese Parametrisierungsphase auf folgende Parameter wirkt :
• die Größe der visuellen Symbole ;
• den Lichtkontrast;
• die Farben;
• die Ereignisdauer und Häufigkeit der Gegenstände und der Symbole ;
• die Zeichen oder die Texte zum Vorlesen: Größe und Abstand der Zeichen und der Zeilen, Inhalt oder Sprache des Textes;
• die Verschiebungsgeschwindigkeit und die Bahn der Gegenstände und der Symbole ;
• das Hintergrundbild : kein Bild, Festbild oder Zeichenbild;
• die Bildverschiebung in den Tests des stereoskopischen Sehens ;
• die Schall- Intensität, -Häufigkeit und -Dauer;
• die Sequenzierung von einzelnen, gleichartigen, modulierten und verketteten Tonfolgen ;
• die ausgesprochenen Wörter: Änderung der Intensität, des Wortschatzes und der Sprache ;
• die Schallverschiebung : Stereophonie ;
• die Geräuscherzeugung im Hintergrund: keine, gleichartige oder modulierte Geräuscherzeugung;
• die gespeicherten akustischen und visuellen Kommentare: sowohl Anweisungen, Fragen und Störgeräusche als auch die Sprache dieser Kommentare.

4. Verfahren nach dem Patentanspruch 2, das **dadurch gekennzeichnet ist, dass** diese Test- oder Übungsplase folgende Punkte betrifft:
• beide Augen gleichzeitig mit derselben Bildsequenz;
• beide Augen gleichzeitig mit einer verschiedenen Bildsequenzen für jedes Auge zum Beispiel mit einem anzuwendenden Hilfsmittel (11), um die Bilder die vom jeden Augen gesehen werden, zu trennen;
• ein Auge nach dem andern mit einer Abdeckung vor dem anderen Auge;
• beide Ohren gleichzeitig mit derselben Schallsequenz;
• beide Ohren gleichzeitig mit einer unterschiedlichen Schallsequenz für jedes Ohr bei Gebrauch eines stereophonischen Kopfhörers oder eines Summers auf dem Mastoid (8) ;
• ein Ohr nach dem ändern unter Zuhilfenahme eines stereophonischen Kopfhörers oder eines Summers auf dem Mastoid (8) ;
• Sehen und Hören zeitlich getrennt oder gleichzeitig: die Studie oder die Stimulation des Gehörs auf Grund der Wahmehmungen und der visuellen Reflexe und umgekehrt; das Gehör, das die Aufmerksamkeit auf einen visuellen Test oder umgekehrt lenken kann.

5. Verfahren nach den Patentansprüchen 2 und 4, das **dadurch gekennzeichnet ist, dass** diese Test- oder Übungsphase auf folgende Bereiche angewendet wird :
• die Sehfunktion ;
• die Hörfunktion ;
• die Schärfe des Fernsehens;
• die Schärfe des Nahsehens;
• die Sehschärfe des Babys ;
• das Sehen der Farben;
• das stereoskopische Sehen mit gefärbten oder polarisierten Gläsern ;
• das binokulare Sehen;
• das Nachtsehen mit möglichem Gebrauch einer Infrarot-Kamera;
• die Studie der Pupillenreflexe ;
• die Ton- und Sprachaudiometrie des Erwachsenen;
• die Ton- und Sprachaudiometrie des Kindes;
• die Studie der Orientierungsreflexe des Babys und des Kindes durch Beobachtung der Bewegungen der Augäpfel und des Kopfes nach die Schallquelle;
• die Identifizierung des 'Recruitment'-Phänomens zum Beispiel mit der Fowler Hörprüfung ;
• die Studie der motorischen Reflexe und der motorischen Reaktionen aus visueller oder akustischer Herkunft.

6. Verfahren nach dem Patentanspruch 2, das **dadurch gekennzeichnet ist, dass** diese Analysephase die chronologische Beobachtung und Analyse in synchronisiertem Bezug auf die Tests oder Übungen folgender Reaktionen des Patienten (1) ermöglicht:
• die Augenbewegungen;
• die Kopfbewegungen ;
• die Hals- und Rumpfbewegungen;
• die Arm- und Handbewegungen;
• die Erweiterung und die Kontraktion der Pupillen;
• der Gesichtsausdruck;
• die Antworten auf die Fragen;
• die Erwähnungen von Gegenständen, Geräuschen, Buchstaben oder Zahlen ;
• das Lesen oder die Wiederholung von Wörtern oder Texten;
• das Zeigen von Gegenständen oder Bildern ;
• die allgemeine Einstellung des Patienten (1) und unter anderem seine Aufmerksamkeitfähigkeiten.

7. Entwicklungsanlage des Verfahrens nach irgendeinem Patentanspruch 1 bis 6, das durch folgende Einrichtung des Materials **gekennzeichnet** ist:
• Nahgeräte: der Bildschirm (3), die Lautsprecher (4), die Kamera (5), das fakultative Mikrophon (6) und das fakultative Zeigegerät (7) sind auf einer höhenverstellbaren Rolltafel auf einer Höhe von ca. 75 cm angeordnet oder mit einem Regelsystem angeordnet, welches den Abstand vom Patienten (1) zwischen ca. 30 und 90 cm auf einem Schiebetablett vor dem Patienten (1) einstellt, der auf einem höhenverstellbaren Stuhl sitzt mit abwechselnd die Lautsprecher (4) und das Mikrophon (6) die auf dem Kopfhörer (8) das vom Patienten (1) getragen wird, gesammelt werden;
• Ferngeräte sind fakultativ : Schirme (10) werden mit Wand- oder Standhalter auf einer Höhe von ca 180 cm und einer Abstand von 3 bis 6 Meter des Patienten (1) festgelegt;
Nach dieser Anordnung steht die Kamera (5) gerade über dem Blick in die Richtung der Nahgeräte (3) und gerade unter dem Blick in die Richtung der fakultativen Ferngeräte (10).
